Europäisches Patentamt

European Patent Office

Office européen des brevets

(19)

(11) Numéro de publication: **0 238 396**
**A1**

## (12) DEMANDE DE BREVET EUROPEEN

(21) Numéro de dépôt: **87400535.8**

(22) Date de dépôt: **11.03.87**

(51) Int. Cl.³: **G 01 N 33/569**
**G 01 N 33/546**

(30) Priorité: **13.03.86 FR 8603572**

(43) Date de publication de la demande:
**23.09.87 Bulletin 87/39**

(84) Etats contractants désignés:
**AT BE CH DE ES FR GB GR IT LI LU NL SE**

(71) Demandeur: **Lurhuma, Zirimwabagabo**
**Cliniques Universitaires de Kinshasa B.P. 123**
**Kinshasa XI(ZR)**

(72) Inventeur: **Lurhuma, Zirimwabagabo**
**Cliniques Universitaires de Kinshasa B.P. 123**
**Kinshasa XI(ZR)**

(74) Mandataire: **Warcoin, Jacques et al,**
**Cabinet Régimbeau 26, avenue Kléber**
**F-75116 Paris(FR)**

(54) **Produit d'immuno-essai, son procédé de préparation, son utilisation, complexe immunogène le comportant et utilisation de ce complexe.**

(57) Produit notamment utile dans les immuno-essais, comportant au moins une particule immunologiquement inerte sur laquelle est fixé au moins un anticorps spécifique d'un antigène particulier.

EP 0 238 396 A1

Croydon Printing Company Ltd.

PRODUIT D'IMMUNO-ESSAI, SON PROCEDE DE PREPARATION,
SON UTILISATION, COMPLEXE IMMUNOGENE LE COMPORTANT
ET UTILISATION DE CE COMPLEXE

La présente invention a pour objet, notamment, un nouveau type d'immuno-essais.

Les immuno-essais ont pour but de mettre en évidence et de doser des anticorps ou des antigènes dans un milieu, notamment dans les milieux physiologiques, et ceci grâce à la capacité d'union spécifique entre les anticorps et les antigènes correspondants.

Dans le cadre des immuno-essais, de nombreuses réactions sont utilisables : immuno-précipitation, immuno-agglutination, réaction de fixation du complément, neutralisation, réaction d'immuno-fluorescence, techniques radio-immunologiques et immuno-enzymatiques.

La présente invention concerne plus particulièrement les réactions d'immuno-agglutination.

Une réaction d'immuno-agglutination est une combinaison entre un antigène et l'anticorps correspondant dont il résulte la formation de gros amas, ou agglutinats.

On distingue la réaction d'agglutination naturelle, dans laquelle l'antigène est particulaire ou figuré, tel qu'une bactérie, de la réaction d'agglutination passive, dans laquelle l'antigène soluble est fixé à la surface de particules figurées immunologiquement inertes.

Ainsi, dans les cas connus jusqu'à présent, la réaction d'agglutination ne permet pas d'aboutir à la détermination d'antigènes solubles.

Par ailleurs, un des objectifs principaux des immuno-essais est le diagnostic sérologique des maladies virales, bactériennes, mycobactériennes ou parasitaires. Ce diagnostic est fondé sur la mise en évidence d'anticorps spécifiques. Or, la présence d'anticorps, même spécifiques, chez un individu ne signifie pas nécessairement que l'agent pathogène est encore présent chez cet individu. En outre, certaines maladies font naître, dans l'organisme, non pas une immunité à médiation humorale qui se manifeste par la formation d'anticorps, mais une immunité à médiation cellulaire ; cette dernière ne génère pas la formation d'anticorps, mais fait intervenir un mécanisme complexe au cours duquel l'antigène se lie à des récepteurs situé sur des cellules spécialisées, les lymphocytes.

En conséquence, de nombreuses maladies ne peuvent pas être dépistées, ou peuvent être dépistées, mais trop tard.

La présente invention se propose, entre autres, de remédier à ces inconvénients en permettant d'établir un diagnostic à partir de la détermination, non pas des anticorps, mais des antigènes. Ainsi, la certitude de la présence desdits antigènes au moment du diagnostic permet de traiter l'individu examiné avec certains médicaments dont on connaît la toxicité et que l'on n'ose pas donner tant que l'on n'est pas sûr du diagnostic.

A cet effet, la présente invention propose un produit comportant au moins une particule immunologiquement inerte sur laquelle est fixé au moins un anticorps spécifique d'un antigène particulier, ledit antigène ne pouvant pas être une toxine. La particule immunologiquement inerte, support de l'anticorps, permet de visualiser la formation de l'agglutination au cours de l'immuno-essai.

Diverses substances peuvent être utilisées à ce titre, à condition, bien sûr, de remplir ce rôle de support et d'être immunologiquement inerte. Elles seront parfois dénommées ci-après "particules-support".

C'est ainsi que certaines matières plastiques, des cristaux de cholestérol, ou même parfois des globules rouges peuvent être utilisés.

En pratique, selon la présente invention, on utilise du polystyrène. On choisit de préférence du latex de polystyrène, c'est-à-dire du polystyrène d'origine synthétique. Parmi ces latex, il faut citer le latex commercialisé par la société SIGMA CORPORATION, le produit dénommé ESTAPOR commercialisé par la société RHONE-POULEC et le latex commercialisé par la société ORTHO PHARMACEUTICAL.

Par ailleurs, à l'issue de différents essais, réalisés avec le produit selon la présente invention, on a constaté que les résultats sont grandement améliorés lorsque l'on fait subir à la particule-support un traitement chimique préalablement à la fixation de l'anticorps.

Ce traitement chimique, ou activation, dépend bien sûr de la nature de la particule retenue.

Dans le cas particulier du latex de polystyrène, le traitement chimique préalable comprend un traitement au glutaraldéhyde, de préférence dans un tampon phosphate (PBS), à un pH de l'ordre de 7,2, cette réaction étant suivie de un ou plusieurs lavages dans le même tampon, puis d'une étape de séparation, de préférence une centrifugation à environ 15 000 tours par minute pendant environ 10 minutes.

4 0238396

A l'issue de chacune de ces deux centrifugations, on élimine le surnageant et on récupère le
précipité.

C'est sur la particule immunologiquement
inerte, de préférence préalablement activée, que l'on
fixe un anticorps spécifique, cette fixation permettant
de générer le produit selon la présente invention.

Les anticorps, ou immunoglobulines, sont
des protéines produites par l'organisme à la suite d'une
stimulation antigénique. De préférence, selon la présente invention, on utilise des immunoglobulines d'origine
animale ou humaine.

Parmi celles-ci, il existe différentes
classes d'immunoglobulines telles que IgG, IgM, IgA,
IgD, IgE.

La classe IgG est la plus courante et
est utilisée de préférence selon la présente invention.

Elle comporte différentes sous-classes,
qui toutes consistent en l'association de deux chaînes
polypeptidiques légères et de deux chaînes polypeptidiques lourdes.

Ces chaînes sont liées entre elles par
des liaisons fortes, ou ponts S——S, et des liaisons faibles, ou liaisons -H-.

Différents clivages enzymatiques d'une
molécule d'IgG ont permis de connaître ses fragments
caractéristiques, schématisés de la façon suivante :

Fragment Fab'₂

Une autre classe d'anticorps utilisés aussi selon la présente invention est la classe IgA car cette classe comporte des anticorps sériques essentiellement antiviraux et antibactériens.

Les anticorps utilisés peuvent être aussi bien d'origine monoclonale que polyclonale.

Le procédé permettant de fixer l'anticorps sur la particule-support varie, bien sûr, en fonction de la nature de cette dernière et de l'anticorps choisi.

Ainsi, dans le cas où la particule inerte est du latex de polystyrène préalablement activé selon le procédé décrit ci-dessus, et dans le cas où l'anticorps est une IgG, on procède, pour sa fixation, de préférence de la façon suivante :

Le précipité de latex de polystyrène activé est incubé dans une solution d'anticorps, en particulier d'IgG, préalablement purifiée, pendant environ 2 jours. Le mélange d'incubation est séparé, par exemple par centrifugation à environ 15 000 tours par minute pendant environ 10 minutes. Le précipité obtenu, débarrassé du surnageant, est mis en suspension dans une solution tampon du type PBS à un pH d'environ 7,2 en présence d'agent tensio-actif tel que Tween 20.

Cet anticorps que l'on choisit de fixer est spécifique d'un antigène particulier caractéristique d'une maladie. De préférence, selon la présente invention, cette maladie est d'origine virale telle que le S.I.D.A. (Syndrome d'Immuno-Déficience Acquise), bactérienne ou mycobactérienne telle que la tuberculose, parasitaire telle que le paludisme ou l'amibiase, voire même mycotique telle que la mycose provoquée par Cryptococcus.

La mise en présence de l'un de ces antigènes avec le produit selon la présente invention, produit comprenant l'anticorps spécifique dudit antigène, conduit à une réaction d'agglutination rapide et visible à l'oeil nu. Une réaction faussement positive peut se produire en présence de certains sérums contenant des auto-anticorps de type IgM. Cette réaction peut être abolie en réduisant ces auto-anticorps, cette réduction n'altère pas les antigènes et donc n'altère pas la réaction spécifique. Il est aussi possible, selon l'invention, d'éliminer le facteur rhumatoïde pouvant donner des faux positifs, en mélangeant des IgG agrégées au sérum à étudier à raison d'environ 60% v/v par rapport audit sérum.

Les IgG sont agrégées par chauffage à une température comprise entre 60°C et 65°C pendant environ 10 minutes, puis concentrées à environ 160 mg/ml.

On peut ainsi envisager de déterminer la nature d'un antigène inconnu à partir d'un anticorps connu. En particulier, la mise en présence du produit selon l'invention et d'un antigène particulier se trouvant dans un milieu physiologique tel que le sérum permet d'aboutir au diagnostic de diverses maladies d'origine virale, bactérienne, mycobactérienne ou parasitaire.

Ainsi, des essais concluants ont été réalisés et ont permis d'aboutir au diagnostic sérologique du S.I.D.A. Le produit selon la présente invention peut donc entrer dans la composition de réactifs de diagnostics, ceux-ci pouvant être préparés sous la forme de kits de diagnostics.

En outre, une réaction d'agglutination peut être quantifiée. On peut donc envisager, non seulement de déterminer la nature d'un antigène, mais aussi de doser ledit antigène.

La présente invention permet également de séparer et purifier une protéine antigénique, évitant ainsi la nécessité de cultures cellulaires longues et coûteuses. En effet, la réaction spécifique d'agglutination permet de sélectionner un antigène donné. Il suffit ensuite de couper, par des techniques connues, la liaison antigène-anticorps pour préparer une protéine antigénique pure.

Des solutions antigéniques pures sont nécessaires dans des cas multiples, notamment lors de la fabrication de vaccins ou pour l'obtention d'anticorps d'origine animale pour la sérothérapie passive.

Enfin, parmi tous les antigènes connus, certains ne sont pas immunogènes, c'est-à-dire qu'ils ne sont pas capables de provoquer la réaction immunitaire, du fait le plus souvent de leur trop faible poids moléculaire.

La réaction d'agglutination à l'aide du produit selon la présente invention permet d'aboutir à la formation d'un complexe antigène-anticorps qui, dans certains cas, confère audit antigène la propriété d'immunogénicité qui lui faisait défaut, grâce notamment à une polymérisation antigénique. Ces antigènes, enfin rendus immunogènes, peuvent entrer dans la composition d'un réactif permettant d'immuniser un organisme vivant, leur injection engendrant la formation d'anticorps spéci-

8   0238396

fiques. La fabrication de vaccins grâce à ces antigènes est donc envisagée, de même que l'obtention d'anticorps d'origine animale pour la sérothérapie passive.

La présente invention sera mieux comprise à la lecture des exemples suivants :

EXEMPLE 1

Procédé de traitement chimique de particules de latex de polystyrène préalablement à la fixation de l'anticorps spécifique

On incube 125 µl d'une suspension de latex de polystyrène à 10% dans 1 ml de tampon PBS contenant 40 µl de glutaraldéhyde à 25%.

Cette incubation dure 30 minutes et se déroule à un pH de 7,2.

Après ce temps d'incubation, on centrifuge à 15 000 tours/minute, pendant 10 minutes. On jette le surnageant, on récupère le précipité. On remet en suspension le précipité dans 2 ml de tampon PBS. On centrifuge à nouveau pendant 10 minutes. On jette le surnageant et on récupère le précipité.

EXEMPLE 2

Procédé de fixation d'IgG sur des particules de latex de polystyrène préalablement activées

Le précipité obtenu à l'exemple 1 est mis en suspension dans 1 ml d'une solution comportant 10 à 20 mg/ml d'IgG spécifiques. Ces IgG ont été préalablement dialysés contre le tampon PBS à un pH de 7,2.

On incube sous agitation pendant 2 jours. Après cette incubation, on centrifuge à 15 000 tours/ minute. On jette le surnageant. On récupère le précipité. On suspend le précipité dans 1 ml de tampon PBS à un pH de 7,2, ce tampon contenant du Tween 20 à la concentration de 0,05% (v/v). Pour permettre la conservation du produit obtenu, on peut ajouter une pincée d'acide sodique $NaN_3$.

EXEMPLE 3

Réaction d'agglutination

20 µl de la préparation obtenue à
l'exemple 2 sont mélangés à 20 µl de sérum contenant
l'antigène à tester. Ce mélange est effectué sur une
plaque à fond noir. On agite. Si le sérum contient les
antigènes spécifiques des anticorps fixés sur le latex,
une agglutination, visible à l'oeil nu, apparaît dans
les 5 minutes qui suivent.

EXEMPLE 4

Certitude du diagnostic

1) Le réactif de diagnostic préparé à
partir du produit selon la présente invention a été testé
en laboratoire et a permis de mettre en évidence des
antigènes sériques solubles dans les cas de tuberculose,
d'amibiase intestinale, de trypanosomiase, de paludisme
et du S.I.D.A. et de diagnostiquer ainsi ces maladies.

Ce réactif a aussi été testé sur le
virus HTLV III/LAV en culture concentrée et sur le surnageant du milieu de culture.

2) La certitude de ce diagnostic a été
établie :

En effet, des sérums contenant le facteur
rhumatoïde peuvent donner des réactions faussement
positives. Mais, il suffit, soit de réduire ces sérums par
du dithiothréitol, soit de préparer de la même façon du
latex recouvert de fragments Fab'$_2$ spécifiques obtenus
par digestion des IgG à la pepsine, pour supprimer tout
risque de réaction faussement positive.

3) Les contrôles :

Les sérums normaux ainsi que le surnageant concentré de culture de cellules sensibles au
HTLV III/LAV non infectées ne donnent pas de réaction
d'agglutination.

**0238396**

EXEMPLE 5

Sérothérapie passive

Le produit préparé selon la présente invention a permis d'aboutir à la production d'anticorps précipitants anti-HTLV III/LAV chez les animaux. On envisage d'administrer ces anticorps aux individus atteints du S.I.D.A. et séro-positifs pour le virus HTLV III/LAV.

EXEMPLE 6

Dans le cadre du vaccin :

Par chromatographie d'affinité (IgG d'un patient séro-positif fixées sur du Sépharose aminé) on isole des sérums de malades une protéine dont la caractérisation est en cours. Cette protéine a été concentrée à 4,6 mg/ml. A un millilitre de cette concentration on ajoute, en agitant 50 microlitres de glutaraldéhyde à 25% dans l'eau puis 1 ml d'anatoxine tétanique concentrée à 4,6 mg/ml. On incube pendant 2 heures à température de labo puis on dialyse longuement contre du PBS pH 7,4.

Pour immuniser on prend 0,5 ml de cette préparation que l'on mélange à 1 ml de phosphate d'alumine et on injecte le tout par voie intramusculaire.

| Jour 0 | Jour 7 | Jour 14 | Jour 21 | Jour 28 |
|--------|--------|---------|---------|---------|
| 1ère piqûre | 2è piqûre | 3è piqûre | 4è piqûre | Saignée |
| | | | | -Saignée |

Après 2 piqûres c'est-à-dire au 14è jour, le mouton présente déjà des anticorps précipitant en réaction d'Ouchterlony et en agglutination de latex recouvert du virus HIV ou de protéines virales (surnageant de culture de cellules infectées par le HIV). Après la 4è piqûre (saignée du jour 28), les anticorps montrent une très grande affinité pour le virus HIV et les antigènes viraux solubles à en juger par la réaction d'agglutination du latex.

Dès la 2ème injection, les anticorps sont précipitants.

Les conditions d'immunisation utilisées ici (par exemple l'adjuvant) sont applicables à l'être humain. Il nous reste à produire ce conjugué en grande quantité et à le stériliser pour l'essayer chez l'homme.

EXEMPLE 7

Le test ELISA pour antigène :

Un antisérum anti-HIV de mouton (IgG) est fixé sur les plaques de microtitrage. Le sérum à tester est mis à incuber avec l'antisérum fixé pendant 1 heure après lavage, des IgG provenant d'un séropositif connu sont incubées avec la plaque pendant encore 1 heure. La plaque est lavée 4 fois puis les IgG humaines fixés sur les antigènes éventuels sont révélées par un antisérum de mouton anti-IgG marqué à la péroxidase.

REVENDICATIONS

1 - Produit utile pour la détermination d'au moins un antigène particulier, à l'exclusion de toxines, lors de réactions d'immuno-essais, caractérisé en ce qu'il comporte au moins une particule immunologiquement inerte sur laquelle est fixé au moins un anticorps spécifique d'un antigène particulier.

2 - Produit selon la revendication 1, caractérisé en ce que la particule immunologiquement inerte est du polystyrène.

3 - Produit selon les revendications 1 et 2, caractérisé en ce que la particule immunologiquement inerte est du latex de polystyrène.

4 - Produit selon l'une des revendications 1 à 3, caractérisé en ce que la particule immunologiquement inerte est activée chimiquement avant la fixation de l'anticorps spécifique.

5 - Produit selon l'une des revendications 1 à 4, caractérisé en ce que l'anticorps est d'origine animale ou humaine.

6 - Produit selon l'une des revendications 1 à 5, caractérisé en ce que l'anticorps est une immunoglobuline appartenant à la classe IgG ou à la classe IgA.

7 - Produit selon la revendication 6, caractérisé en ce que l'anticorps utilisé est une IgG.

8 - Produit selon l'une des revendications 1 à 7, caractérisé en ce que l'antigène particulier est caractéristique d'une maladie d'origine virale, bactérienne, mycobactérienne ou parasitaire.

9 - Produit selon la revendication 8, caractérisé en ce que l'antigène est caractéristique du S.I.D.A.

10 - Procédé d'activation chimique de la particule immunologiquement inerte selon les revendications 3 et 4, caractérisé en ce qu'il comporte un traitement au glutaraldéhyde, de préférence dans une solution tampon à un pH d'environ 7,2, ce traitement étant suivi d'un ou plusieurs lavages dans le même tampon et d'une étape de séparation à l'issue de laquelle le liquide surnageant est éliminé et le précipité est récupéré.

11 - Procédé de fixation de l'anticorps selon la revendication 7 sur la particule immunologiquement inerte, préalablement activée selon la revendication 10, caractérisé en ce que le précipité obtenu est incubé dans une solution d'IgG pendant environ 2 jours, et en ce que cette incubation est suivie d'une étape de séparation à l'issue de laquelle le précipité récupéré est mis en suspension en présence d'un agent tensio-actif dans une solution tampon à un pH d'environ 7,2.

12 - Utilisation du produit selon l'une des revendications 1 à 9 pour la préparation d'agents permettant la détermination, le dosage, la séparation ou la purification d'un antigène particulier.

13 - Utilisation du produit selon la revendication 12, caractérisée en ce que l'antigène particulier se trouve dans un milieu physiologique.

14 - Utilisation du produit selon la revendication 12 ou 13 pour la préparation d'un réactif permettant le diagnostic de maladies bactériennes, virales mycobactériennes ou parasitaires.

15 - Utilisation du produit selon la revendication 14 pour la préparation d'un réactif permettant le diagnostic du S.I.D.A.

16 - Utilisation du produit selon la revendication 12 pour la préparation de protéines antigéniques pures.

17 - Utilisation des antigènes selon la revendication 16 pour la préparation de vaccins pour l'obtention d'anticorps d'origine animale.

18 - Complexe immunogène caractérisé en ce qu'il comporte le produit selon l'une des revendications 1 à 9 auquel s'est aggluté l'antigène spécifique de l'anticorps dudit produit.

19 - Utilisation du complexe selon la revendication 18 pour la préparation de vaccins ou pour l'obtention d'anticorps d'origine animale.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int Cl.4) |
|---|---|---|---|
| X | DE-A-2 529 937 (TAKEDA CHEMICAL INDUSTRIES)<br>* Revendications 1-4,15,16,20-25; page 17, lignes 5-22 * | 1-8,11 -14,18 | G 01 N 33/569<br>G 01 N 33/546 |
| | --- | | |
| X | DE-A-2 746 101 (AMERICAN CYANAMID CO.)<br>* Revendications; page 6, lignes 23-31; page 7, lignes 1-9 * | 1-8,11 -14,18 | |
| | --- | | |
| X | EP-A-0 000 772 (HOFFMANN-LA ROCHE & CO.)<br>* Revendications 1,2,5-16; page 18, lignes 1-37; page 4, lignes 5-37; page 5, ligne 15; page 6 * | 1-8,10 -14,18 | |
| | --- | | |
| Y | EP-A-0 135 352 (FUJIREBIO KABUSHIKI KAISHA)<br>* Abrégé; revendications 1,7 * | 1,8,9 14,15 | DOMAINES TECHNIQUES RECHERCHES (Int. Cl.4) |
| | --- | | |
| Y | THE LANCET, no. 8389, 9 juin 1984, pages 1253-1256, The Lancet Ltd, Londres, GB; F. BRUN-VEZINET et al.: "Detection of IgG antibodies to lymphadenopathy-associated virus in patients with aids or lymphadenopathy syndrome"<br>* Page 1253, abrégé, introduction * | 1,8,9 14,15 | G 01 N |
| | ---     -/- | | |

Le présent rapport de recherche a été établi pour toutes les revendications

| Lieu de la recherche | Date d'achèvement de la recherche | Examinateur |
|---|---|---|
| LA HAYE | 16-06-1987 | MEYLAERTS H. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82

### Office européen des brevets

# RAPPORT DE RECHERCHE EUROPEENNE

Numero de la demande

# 0238396

EP  87 40 0535

| | DOCUMENTS CONSIDERES COMME PERTINENTS | | Page  2 |
|---|---|---|---|
| **Catégorie** | Citation du document avec indication, en cas de besoin, des parties pertinentes | Revendication concernée | CLASSEMENT DE LA DEMANDE (Int. Cl.4) |
| X | EP-A-0 101 166  (FUJIREBIO KABUSHIKI KAISHA)<br>* Abrégé; page 1, lignes 1-7; page 3- page 4; page 5, lignes 4-17; revendications 1,2,4,5,8 *<br><br>--- | 1-15 | |
| X | EP-A-0 121 243  (MITSUBISHI CHEMICAL INDUSTRIES LTD)<br>* Revendications 1-4 *<br><br>--- | 1-8 | |
| A | WO-A-8 402 194  (THE ROCKEFELLER UNIVERSITY)<br>* Page 1, lignes 1-10 *<br><br>--- | 6 | |
| A | WO-A-8 404 327  (PRESIDENT AND FELLOWS OF HARVARD COLLEGE)<br>* Revendications 1,10,11 *<br><br>----- | 9,15 | DOMAINES TECHNIQUES RECHERCHES (Int. Cl.4) |

Le présent rapport de recherche a été établi pour toutes les revendications

| Lieu de la recherche | Date d'achèvement de la recherche | Examinateur |
|---|---|---|
| LA HAYE | 16-06-1987 | MEYLAERTS H. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82